# EUROPÄISCHE PATENTSCHRIFT

(11) **EP 2 257 235 B1**
(45) Veröffentlichungstag und Bekanntmachung des Hinweises auf die Patenterteilung: **29.04.2015**
(21) Anmeldenummer: 09711752.7
(22) Anmeldetag: 11.02.2009
(51) Int. Cl.: A61B 18/02

(54) **KRYOCHIRURGISCHES INSTRUMENT**
CRYOSURGICAL INSTRUMENT
INSTRUMENT CRYOCHIRURGICAL

(30) Priorität: 21.02.2008 DE 102008010477
(43) Veröffentlichungstag der Anmeldung: 08.12.2010
(73) Patentinhaber: ERBE Elektromedizin GmbH, 72072 Tübingen (DE)
(72) Erfinder: GEISELHART, Franz, 72770 Reutlingen (DE)
(74) Vertreter: Bohnenberger, Johannes
(86) Internationale Anmeldenummer: PCT/EP2009/000956
(87) Internationale Veröffentlichungsnummer: WO 2009/103448

(56) Entgegenhaltungen:
- WO-A-99/04211
- WO-A-2005/000106
- US-A1- 2003 023 288
- US-A1- 2004 054 361
- US-B1- 6 270 493

## Beschreibung

Vorliegende Erfindung betrifft ein kryochirurgisches Instrument, umfassend eine Kryosonde mit einem von einem komprimierten Kühlfluid, insbesondere Kühlgas durchströmten Leitungssystem, zum Abkühlen wenigstens eines Teils der Kryosonde.

In der Kryochirurgie wird die gezielte, kontrollierte Kälteanwendung zur Devitalisierung von biologischem Gewebe eingesetzt. Insbesondere mit flexiblen Sonden werden zudem Fremdkörper aus Körperhöhlen durch Festfrieren an der Kryosonde bzw. an einem Sondenkopf extrahiert, so z.B. verschluckte und dabei versehentlich eingeatmete Fremdkörper, die aus den Atemwegen entfernt werden müssen. Die Kryochirurgie eignet sich aber auch zur Gewinnung von Gewebeproben (Biopsie). Dabei friert ein bestimmter Gewebebereich, die Gewebeprobe, an den Sondenkopf an, und kann nach dem Abtrennen von umliegendem Gewebe einer Untersuchung zugänglich gemacht werden.

Um in der Chirurgie tiefzufrieren, gibt es verschiedene Möglichkeiten. Eine stützt sich auf den Joule-Thomson-Effekt: die Atome bzw. Moleküle eines sich expandierenden Fluides und insbesondere Gases unterhalb der Inversionstemperatur arbeiten gegen die gegenseitige Anziehung an, so dass das Gas an innerer Energie verliert. Es kühlt ab. Als expandierendes Fluid bzw. Gas - im Folgenden Kühlfluid bzw. Kühlgas genannt - werden üblicherweise CO₂ oder N₂O eingesetzt.

Kryochirurgische Instrumente der eben beschriebenen Art verfügen üblicherweise über eine Sonde, die an das zu behandelnde Gewebe verbracht werden kann, ferner über Gasleitungseinrichtungen, welche die Sonden durchsetzen und innerhalb der Sonden das Arbeitsgas in das Innenvolumen der Sonden entlassen, wo es expandiert und in Folge dessen die Sonde abkühlt. Da diese vorzugsweise aus thermisch leitfähigem Material gefertigt ist, ist ein Ableiten der Gewebewärme über die Sonde und damit eine Kühlwirkung gewährleistet.

Bei Kryosonden, bei denen die Kühlwirkung wie oben beschrieben durch Entspannung komprimierter Gase erzeugt wird, besteht häufig die Anforderung, eine größere Oberfläche gleichmäßig oder nach einem bestimmten Temperaturprofil zu kühlen. Beispielsweise soll eine Kryosonde von 2 mm Durchmesser über eine Länge von 50 mm gleichmäßig gekühlt werden.

Nach dem Stand der Technik werden in solchen Fällen mehrere Düsen im Inneren der Kryosonde verteilt angeordnet, um eine einigermaßen gleichmäßige Kühlung zu erreichen. Je größer die Anzahl der verwendeten Kühldüsen ist, desto gleichmäßiger stellt sich die resultierende Temperatur über die gewählte Länge ein. Bei dieser Vorgehensweise wird die benötigte Gasmenge auf mehrere Düsen verteilt. Die einzelnen Düsen sind folglich in ihrem Querschnitt sehr klein. Gleichzeitig wird die Herstellung aber überproportional aufwändig. Bei kleinen Düsen werden an die Geometrie enge Toleranzen gestellt, um ein gleich bleibendes Strömungsverhalten erzielen zu können. Kleinere Düsenquerschnitte sind auch prinzipiell anfälliger für Verstopfungen.

Problematisch hat sich bei kryochirurgischen Instrumenten nach dem Stand der Technik herausgestellt, dass die Realisierung eines im Wesentlichen gleich bleibenden Temperaturverlaufs oder eines vorbestimmten Temperaturverlaufs nur sehr schwer möglich ist. Darüber hinaus ist der Aufbau der herkömmlichen, aus dem Stand der Technik bekannten Kryosonden sehr aufwändig und kostenintensiv.

In WO 2005/000106 A ist ein kryochirurgisches Instrument umfassend ein von einem komprimierten Kühlfluid durchströmbares Leitungssystem zum Abkühlen eines Teils des Instruments offenbart.

Der vorliegenden Erfindung liegt folglich die Aufgabe zu Grunde, ein kryochirurgisches Instrument der eingangs genannten Art dahin gehend weiter zu bilden, dass eine technisch einfacher zu realisierende und verbesserte, insbesondere gleichmäßigere Kühlung der Kryosonde erreichbar ist.

Diese Aufgabe wird durch ein kryochirurgisches Instrument gemäß Patentanspruch 1 gelöst.

Insbesondere wird diese Aufgabe durch ein kryochirurgisches Instrument, umfassend eine Kryosonde, mit einem von einem komprimierten Kühlfluid, insbesondere Kühlgas durchströmten Leitungssystem, zum Abkühlen wenigstens eines Teils der Kryosonde, dadurch gelöst, dass das Leitungssystem einen Expansionskanal aufweist, der einen sich in Strömungsrichtung über eine vorbestimmte Länge sukzessiv vergrößernden Leitungsquerschnitt derart aufweist, dass sich das komprimierte Kühlgas auf seinem Strömungsweg durch den Expansionskanal sukzessive über die vorbestimmte Länge hinweg bei gleichzeitiger Abkühlung wenigstens teilweise entspannt.

Der Kern der Erfindung liegt darin, einen über eine vorbestimmte Länge verlaufenden Kanal so zu gestalten, dass sein Querschnitt über diese Länge derart zunimmt, dass sich die Expansion des Gases über diese Länge des Kanals verteilt. Somit kommt es am Anfang des Kanals nur zu einer Teil-Expansion und somit nicht zur vollen Temperaturdifferenz, durch die sukzessive Expansion über die gesamte Länge jedoch zu einer Abkühlung über einen großen Bereich, wobei die erreichte Temperatur an der Außenseite der Sonde bzw. deren Verteilung u.a. von der jeweiligen Querschnittserweiterung und dem Wärmeübergangswiderstand zwischen dem Expansionskanal und der Außenseite der Kryosonde abhängt.

Vorzugsweise steht der Expansionskanal dabei über einen Wärmeleiter mit der Außenseite der Kryosonde in wärmeleitfähiger Verbindung. Als Wärmeleiter können entweder eigenständige Bauteile mit einem reduzierten Wärmeübergangswiderstand, oder aber entsprechende Hilfsmittel, wie Wärmeleitpasten etc. verwendet werden. Darüber hinaus ist es natürlich auch möglich, ein Gehäusebauteil der Kryosonde entsprechend als Wärmeleiter auszubilden und aus einem Material auszubilden, das eine optimale Wärmeüberleitung garantiert. Vorzugswiese ist der Expansionskanal möglichst nahe der Außenseite der Kryosonde bzw. nahe den Bereichen angeordnet, die gekühlt werden sollen.

Vorzugsweise weist der Expansionskanal einen in Abhängigkeit eines Wärmeübergangswiderstandes des Wärmeleiters derart ausgebildeten Leitungsquerschnitt auf, dass sich auf der Außenseite der Kryosonde wenigstens über einen durch die oben genannte vorbestimmte Länge definierten Teilbereich eine vorbestimmte Temperaturverteilung und insbesondere eine gleich bleibende Temperatur einstellt. Das bedeutet, dass beispielsweise in sehr dicken Wandbereichen zwischen Expansionskanal und Außenseite bzw. einem zu kühlendem Bereich der Expansionskanal entsprechend stark erweitert ausgebildet wird, um hier eine besonders effektive Expansion und somit Abkühlung zu erzielen. Auch ist es natürlich möglich, in Bereichen auf der Außenseite der Kryosonde, bei denen eine sehr starke Abkühlung gewünscht ist, entsprechende Anpassungen am Expansionskanal vorzunehmen. Durch eine gezielte Anpassung des Querschnitts des Expansionskanals kann so, unter Beachtung beispielsweise der vorhandenen Wanddicken zur Außenseite, der Strömungsgeschwindigkeit etc., eine gleichmäßige oder jede andere gewünschte Temperaturverteilung auf der Außenseite der Kryosonde erreicht werden.

Vorzugsweise ist der Expansionskanal derart ausgebildet, dass das Kühlgas in einer turbulenten Strömung strömt. Hier von Bedeutung ist u.a. natürlich der Strömungswiderstand im Expansionskanal, der Expansionskanalquerschnitt und darüber hinaus aber auch der Kühlfluiddruck, der insbesondere durch eine externe Kühlfluidversorgung bereitgestellt wird. Der Vorteil der turbulenten Strömung im Expansionskanal ist, dass es zu einer sehr effektiven Wärmeableitung und somit zu einer verbesserten Kühlung der Außenseite der Kryosonde kommt.

Vorzugsweise ist in wenigstens einem Teilbereich des Leitungssystems zur Bildung des Expansionskanals mindestens ein insbesondere sich in Strömungsrichtung verjüngendes/erweiterndes Modellierungsbauteil derart angeordnet, dass sich der resultierende Leitungsquerschnitt in Strömungsrichtung sukzessive vergrößert. Ist das Leitungssystem ein von Seitenwänden begrenzter Kanal, so kann das Modellierungsbauteil als ein sich in Strömungsrichtung verjüngendes insbesondere keilförmiges Bauteil an wenigstens einer dieser Seitenwände angeordnet werden, wodurch sich eben ein solcher erweiternder Expansionskanal ergibt, durch den das Kühlfluid bei gleichzeitiger sukzessiver Entspannung und Abkühlung strömen kann. An Stelle des zuvor erwähnten sich verjüngenden, keilförmigen Bauteils ist es auch möglich, ein Modellierungsbauteil in Form eines Hohlkörpers zu verwenden, beispielsweise ein Bauteil mit einer im Wesentlichen zentrischen Bohrung, dessen Bohrungswandung sich in Strömungsrichtung sukzessive erweitert, was ebenfalls zu einem sich erweiternden Expansionskanal führt.

Vorzugsweise wird der Expansionskanal durch ein Rohr und ein Modellierungsbauteil gebildet, das als ein sich kegelstumpf oder dergleichen rotationssymmetrisches, sich verjüngendes Bauteil in das Rohr eingesetzt ist. Je nach gewünschter Temperaturverteilung an der Außenseite der Kryosonde kann also auch hier das Modellierungsbauteil in seiner Form verändert und somit direkt auf die Kühlwirkung Einfluss genommen werden. In diesem Zusammenhang ist es natürlich auch möglich, in das Rohr ein Modellierungsbauteil in Form eines Hohlkörpers einzusetzen, dessen Hohlkörperwandungen in Strömungsrichtung sukzessive erweiternd ausgebildet sind, so dass sich ebenfalls ein erweiternder Expansionskanal ergibt. Dies entspricht im Wesentlichen der zuvor genannten Ausführungsform des im Kanal angeordneten, sich erweiternden Hohlkörperbauteils und insbesondere eines rotationssymmetrischen Hohlkörpers als Modellierungsbauteil.

Vorzugsweise bildet das Rohr wenigstens teilweise ein Kryosondengehäuse der Kryosonde, wobei das Rohr vorzugsweise aus einem Material besteht, dass eine gute Wärmeleitfähigkeit aufweist. Eine solche Ausführung der Kryosonde erlaubt die sehr einfache und kostengünstige Herstellung der Kryosonde gemäß den zuvor genannten Bauformen.

Selbstverständlich ist es möglich, an Stelle der zuvor genannten sich verjüngenden oder erweiternden Modellierungsbauteile, entsprechend andere Modellierungsbauteile oder Modelierungsbauteilgruppen zu verwenden, um einen sich in Strömungsrichtung sukzessive vergrößernden Querschnitt des Leitungssystems und somit einen Expansionskanal zur Abkühlung der Außenseite der Kryosonde herzustellen.

Das Rohr umfasst vorzugsweise ein Innengewinde, und das Modellierungsbauteil auf einer sich verjüngenden Wandung ein komplementäres Außengewinde, mit dem es in das Rohr unter Bildung eines sich in Strömungsrichtung sukzessive vergrößernden Expansionskanals im resultierenden Gewindegang einschraubbar ist. Dadurch, dass der Expansionskanal im Wesentlichen wendelförmig um das Modellierungsbauteil herumläuft, vergrößert sich der Expansionsweg und somit die zu erzielende maximale Kühlleistung. Darüber hinaus ermöglicht eine solche Ausführungsform die sehr viel flächendeckendere Kühlung der Kryosonde. Natürlich kann durch eine geeignete Wahl unterschiedlicher Gewindearten auch auf die resultierende Außentemperatur bzw. deren Verteilung Einfluss genommen werden.

Vorzugsweise umfasst das Rohr ein Innengewinde und das Modellierungsbauteil ein komplementäres Außengewinde, mit dem es in das Rohr unter Bildung des Expansionskanals im resultierenden Gewindegang einschraubbar ist, wobei nun das Außengewinde als ein in Strömungsrichtung verlaufendes kegeliges oder dergleichen Gewinde und/oder das Innengewinde als ein in entgegengesetzter Richtung verlaufendes kegeliges oder dergleichen Gewinde ausgebildet ist. Unter kegeliges Gewinde sind hier Gewinde zu verstehen, deren Gewindegangtiefe sukzessive zunimmt, ähnlich der Außengeometrie eines Kegels. Beim Einschrauben in ein komplementäres Außen- bzw. Innengewinde entsteht so ein in Strömungsrichtung im Querschnitt expandierender Expansionskanal.

Anstelle der zuvor beschriebene Gewinde jeglicher Bauart, können natürlich auch vergleichbare Kanäle, Nuten etc. im Rohr- oder Modellierungsbauteil ausgebildet werden, um einen entsprechenden Expansionskanals zu bilden. Hier sind sämtliche aus dem Stand der Technik bekannte Verfahren Bildung von Strömungskanälen etc. anwendbar.

Vorzugsweise wird der Expansionskanal zwischen einem äußeren Rohr und einem darin verlaufenden inneren Rohr gebildet. Das hat zur Folge, dass durch das innere Rohr das wärmere Kühlfluid zugeführt werden kann, um im weiter außen gelegenen Expansionskanal zu expandieren und die Außenseite des äußeren Rohres effektiv zu kühlen. In diesem Zusammenhang kann dann das äußere Rohr als Kryosondengehäuse verwendet werden oder über einen entsprechenden Wärmeleiter mit einem solchen Gehäuse verbunden sein.

Zur Bildung des sich sukzessive erweiternden Expansionskanals im doppelwandigen Rohr können sämtliche zuvor bereits abgehandelten Möglichkeiten herangezogen werden, wobei das Modellierungsbauteil dann vorzugsweise in den Außenkanal einsetzbar oder darin integriert ausgebildet ist. In diesem Zusammenhang ist es auch möglich ein Hohlkörperbauteil als Modellierungsbauteil zu verwenden, wobei dessen Hohlkörperbereich als insbesondere mittig angeordneter Zulauf fungiert.

Vorzugsweise umfasst die Kryosonde eine Kryosondenspitze, die an einem Rohrende des zuvor beschriebenen doppelwandigen Rohres angeordnet ist und das Innenrohr mit dem Expansionskanal unter Bildung eines Umlenkkanals in Fluidverbindung bringt. Auf diese Weise können das innere Rohr und das äußere Rohr bzw. der im äußeren Rohr gebildete Außenkanal zur Zu- und Ableitung des Kühlfluides insbesondere -gases verwendet werden. Die resultierende geometrische Außenform eines solchen Rohres entspricht einer Kanüle und somit einer für die Kryochirurgie sehr anwendungsfreundlichen Form.

Vorzugsweise ist die Kryosondenspitze derart am Rohrende angeordnet und insbesondere über eine Gewindeeinrichtung ein- und ausschraubbar, dass der Querschnitt des Umlenkkanals veränderbar und insbesondere an einen Einlaufbereich des Expansionskanals adaptierbar ist. Der Vorteil liegt darin, dass nun durch eine Adaption des Umlenkkanals auch im Spitzenbereich der Kryosonde auf eine mögliche Expansion des Kühlfluidgases Einfluss genommen werden kann.

Vorzugsweise ist um das innere Rohr eines wie oben beschriebenen doppelwandig ausgeführten Rohres ein in Strömungsrichtung wendelförmig, mit ansteigender Steigung verlaufendes Modellierungsbauteil, insbesondere ein Draht angeordnet, der mit den Wandungen beider Rohre jeweils in fluiddichter Verbindung steht, so dass zwischen benachbarten Windungen und den Wandungen der Rohre ein wendelförmiger, im Querschnitt sukzessive zunehmender Expansionskanal gebildet wird. Natürlich kann auch durch die Wahl des verwendeten, umlaufenden Modellierungsbauteils auf die Geometrie und den Querschnitt des Expansionskanals Einfluss genommen werden. So ist es beispielsweise möglich, mit einem sich sukzessive verjüngenden Modellierungsbauteil, das mit einer gleichmäßigen Wendelsteigung auf das innere Rohr aufgebracht wird, einen sich sukzessive erweiternden Expansionskanal herzustellen.

Vorzugsweise weist grundsätzlich das Modellierungsbauteil, insbesondere im nach Außen weisenden Bereich eine gute Wärmeleitfähigkeit auf, um eine effektive Kühlung der Außenseite bzw. der zu kühlenden Bereiche der Kryosonde sicherzustellen. Um eine effektive Abkühlung der Außenseite zu erzielen ist es zudem möglich, im nach innen weisen Bereich, also beispielsweise dem Bereich, der an den Zulauf mit dem "warmen" Kühlfluid vor seiner Entspannung grenzt, entsprechende Dämmschichten anzuordnen. Dies kann wieder durch die Verwendung entsprechender dämmender Zwischenschichten oder durch eine entsprechende Materialwahl des Modellierungsbauteils oder des inneren Rohres etc. erreicht werden.

Grundsätzlich ist es möglich, das wendelförmige Modellierungsbauteil auf das innere Rohr aufzuwickeln, oder aber auch in Form einer eigenständigen Wendel in den Zwischenraum zwischen Innenrohr und äußerem Rohr einzubringen und insbesondere einzudrehen.

Vorzugsweise ist das Modellierungsbauteil integral am inneren Rohr und/oder am äußeren Rohr und insbesondere durch eine wendelförmige Einkerbung der jeweiligen Wandungen des Rohres gebildet, wobei in Strömungsrichtung die Steigung der wendelförmigen Einkerbung und/oder deren Breite zunimmt. Auch auf diese Weise kann also ein Expansionskanal geschaffen werden, der wendelförmig das innere Rohr umläuft und derart im Querschnitt erweitert ausgebildet ist, dass es zu einer sukzessiven Entspannung des Gases und somit zu einer Abkühlung über die gesamte Länge des Expansionskanals kommt. Natürlich ist es hier möglich, über entsprechende Querschnittsänderung über die Länge des Expansionskanals im Vergleich kältere und weniger kältere Bereiche zu schaffen, wenn dies nötig ist. Auch ist es natürlich möglich, die integral am inneren Rohr und/oder am äußeren Rohr ausgebildeten Modellierungsbauteile durch Ausfräsungen der benachbarten Gebiete auszubilden, die dann passgenau an der komplementären Rohrwand anstehen etc. Auch ist es möglich, an einem oder an beiden Rohren entsprechende Führungsnuten oder -einrichtungen anzuordnen, die das Einsetzten eines wendelförmigen Modellierungsbauteils erlauben. Auch können hier natürlich eine Mehrzahl an unterschiedlichen Nuten vorgesehen sein, um beispielsweise unterschiedliche Modellierungsbauteile einzufügen oder aber auch unterschiedliche Steigungen, Querschnittsänderungen etc. zu erzielen.

Im Zusammenhang mit Modellierungsbauteilen, die in das Leitungssystem und insbesondere in den Außenkanal eines doppelwandigen Rohres einführbar sind, ist es auch denkbar, das Modellierungsbauteil derart flexibel auszubilden, dass der Expansionskanal nach Bedarf veränderbar ist. So kann beispielsweise durch das Einführen einer auf das Innenrohr passgenau aufsteckbaren elastischen Wendel mit in Strömungsrichtung ansteigendem Gewinde, die ebenfalls passgenau an der Wandung des Außenrohrs ansteht, durch ein Verschieben bzw. verdrehen der Wendel in Axialrichtung des Rohres die Querschnittsveränderung des Expansionskanals beeinflusst werden.

Eine Anpassung kann natürlich auch über mehrteilige Modellierungsbauteile erfolgen, die eine Veränderung des Querschnitts des Expansionskanals über seine Länge zulassen.

Vorzugsweise ist diese Anpassung über einen Griff am Kryosondengerät oder eine externe Steuereinrichtung möglich, um auch während der Operation die Temperatur der Kryosonde regeln zu können. Hier sind sämtliche aus dem Stand der Technik bekannte Verfahren zur Steuerung eines chirurgischen Gerätes anwendbar.

Weitere Ausführungsformen der Erfindung ergeben sich aus den Unteransprüchen.

Im Folgenden wird die Erfindung anhand von acht Ausführungsbeispielen beschrieben, die durch die beiliegenden Zeichnungen näher erläutert sind. Hierbei zeigen:
- - Fig. 1: eine schematische Darstellung des erfindungsgemäßen Kryochirurgiegerätes;
- - Fig. 2: eine erste Ausführungsform einer Kryosonde des Kryochirurgiegerätes aus Fig. 1;
- - Fig. 3: eine zweite Ausführungsform einer Kryosonde des Kryochirurgiegerätes aus Fig. 1;
- - Fig. 4: eine dritte Ausführungsform einer Kryosonde des Kryochirurgiegerätes aus Fig. 1;
- - Fig. 5: eine vierte Ausführungsform einer Kryosonde des Kryochirurgiegerätes aus Fig. 1;
- - Fig. 6: eine fünfte Ausführungsform einer Kryosonde des Kryochirurgiegerätes aus Fig. 1;
- - Fig. 7: eine sechste Ausführungsform einer Kryosonde des Kryochirurgiegerätes aus Fig. 1;
- - Fig. 8: eine siebte Ausführungsform einer Kryosonde des Kryochirurgiegerätes aus Fig. 1;
- - Fig. 9: eine achte Ausführungsform einer Kryosonde des Kryochirurgiegerätes aus Fig. 1; und
- - Fig. 10: eine weitere Ausführungsform.

Im Folgenden werden für gleiche und gleich wirkende Bauteile dieselben Bezugszeichen verwendet.

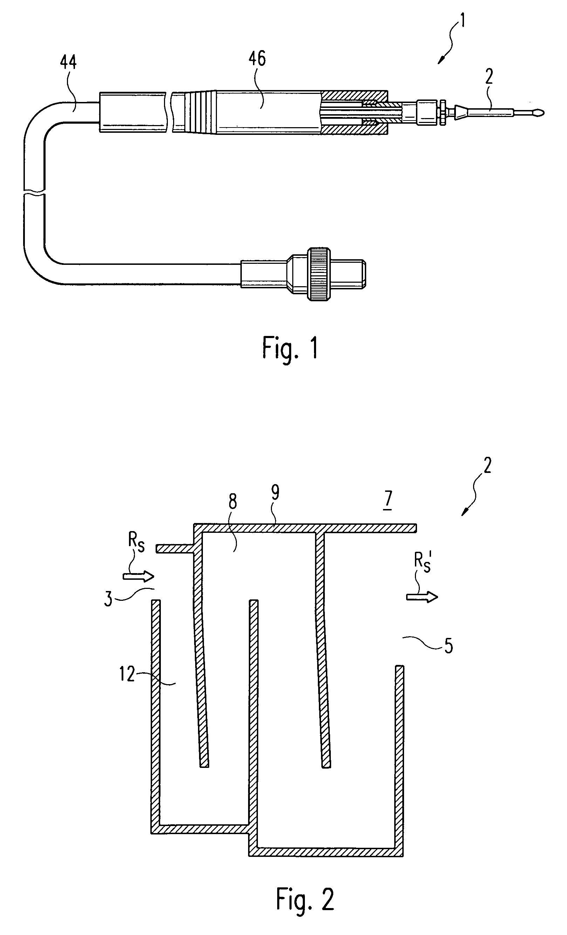

Fig. 1 zeigt eine isometrische Darstellung eines kryochirurgischen Instruments gemäß der vorliegenden Anmeldung. Das Instrument 1 besteht aus einem Kryosondenhalter 46 und einer darin einsetzbaren Kryosonde 2, die erfindungsgemäß durch eine im Folgenden noch detailliert beschriebene Ausbildung eines Leitungssystems derart kühlbar ist, dass kryochirurgische Operationen damit durchgeführt werden können.

Das kryochirurgische Gerät ist mit Gaszuleitungseinrichtungen 44 ausgerüstet, die den Anschluss an ein Gasreservoir (nicht dargestellt) oder eine ähnliche Gasversorgung ermöglichen.

In Fig. 2 ist eine schematische Darstellung einer ersten Ausführungsform der Kryosonde 2 zu sehen. Diese ist hier im Wesentlichen als eine Kryosondenfläche ausgebildet, die ein im Wesentlichen flächig, mäanderndes Leitungssystem 8 aufweist, das mit einem Kühlgas in einer Strömungsrichtung R_{S} durchströmt wird.

Das Leitungssystem 8 ist dabei derart ausgebildet, dass es einen sich über den Strömungsweg entlang der Strömungsrichtung R_{S} erweiternden Leitungsquerschnitt aufweist und so einen Expansionskanal 12 bildet. Das hat zur Folge, dass sich das über den Zulauf 3 einströmende Kühlgas auf seinem Weg durch den Expansionskanal 12 zu einem Ablauf 5 sukzessive entspannt und so gemäß dem Joule-Thomson-Effekt abkühlt. Durch eine entsprechend wärmeleitfähige Ausbildung der Kanalwandungen 9 des Leitungssystems 8 kann so eine Außenseite 7 der Kryosonde 2 effektiv gekühlt werden.

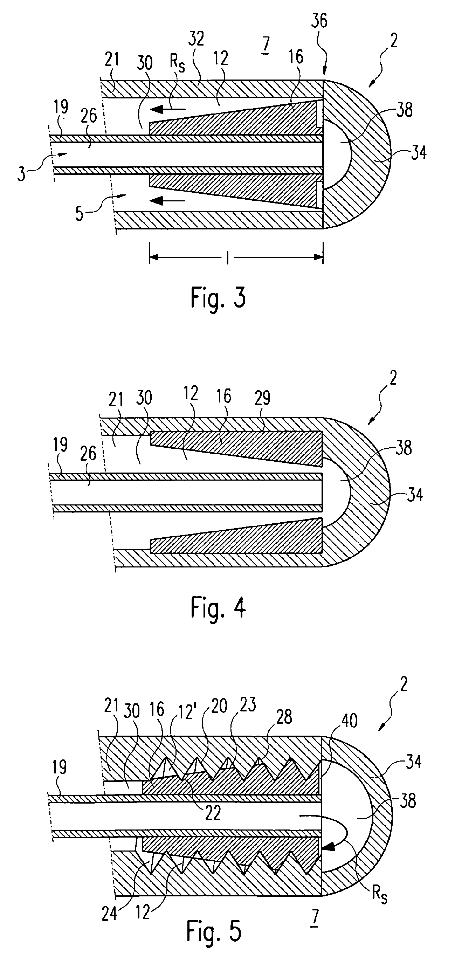

Die Fig. 3-9 zeigen weitere Ausführungsformen der erfindungsgemäßen Kryosonde 2, wobei diese hier grundsätzlich aus einem doppelwandigen Rohr, umfassend ein inneres Rohr 19 und ein äußeres Rohr 21, gebildet werden. Das innere Rohr 19 fungiert dabei als Zulauf 3, über den ein Kühlfluid durch die Kryosonde 2 geleitet werden kann. Am freien Ende 36 ist eine Kryosondenspitze 34 angeordnet, die unter Bildung eines Umlenkkanals 38 eine Verbindung zwischen dem inneren Kanal 26 des Innenrohres 19 und dem äußeren Kanal 30 herstellt, der durch das innere Rohr 19 und das äußere Rohr 21 gebildet wird.

In diesem äußeren Kanal 30 ist ein Modellierungsbauteil 16 angeordnet, das sich in Strömungsrichtung R_{S} derart verjüngt, dass sich ein über eine Länge l sukzessive erweiternder Expansionskanal 12 bildet. Kühlfluid, das über den Zulauf 3 durch den inneren Kanal 26, von dort über den Umlenkkanal 38 in den Expansionskanal 12 strömt, expandiert dort sukzessive über die Länge l, so dass es zu dessen Abkühlung und somit über die wärmeleitende äußere Wandung 32 des äußeren Kanals 30 zu einer Abkühlung auf der Außenseite 7 der Kühlsonde 2 kommt.

Da die hier abgebildete Kryosonde 2 rotationssymmetrisch aus einem doppelwandigen Rohr mit einer gleichbleibend dicken äußeren Wandung 32 und einem als Modellierungsbauteil 12 entsprechend rotationssymmetrischem, sich verjüngendem Hohlkörper ausgebildet ist, kommt es auch zu einer gleichmäßigen Abkühlung über den Umfang der Kryosonde 2. Natürlich ist es auch möglich, durch eine entsprechend veränderte Ausführung des Modellierungsbauteils 16 oder der Lage des inneren Rohres 19 relativ zum äußeren Rohr 21, eine ungleichmäßige Kühlung über den Umfang der Kryosonde 2 zu erzielen.

Fig. 4 zeigt eine dritte Ausführungsform der Kryosonde 2, wieder umfassend ein inneres Rohr 19 und ein äußeres Rohr 21, deren Kanäle 26 bzw. 30 durch eine Kryosondenspitze 34 und einen daraus resultierenden Umlenkkanal 38 miteinander in Fluidverbindung stehen. Auch hier ist im äußeren Kanal 30 ein Modellierungsbauteil 16 zur Bildung eines Expansionskanals 12 eingesetzt. Das Modellierungsbauteil 16 ist hier als ein rotationssymmetrischer Hohlzylinder mit sich sukzessive erweiternden Innenwänden ausgebildet. Um den Hohlzylinder 16 in seiner Lage innerhalb des äußeren Rohres 21 zu fixieren, weist dieses Einsetznuten 29 auf, die das passgenaue Einsetzen erlauben.

Fig. 5 zeigt eine vierte Ausführungsform der Kryosondenspitze 2, wieder bestehend aus einem doppelwandigen Rohr 19, 21, in dessen Außenkanal 30 zur Bildung des Expansionskanals 12 ein Modellierungsbauteil 16 eingesetzt ist.

Im Unterschied zu den zuvor beschriebenen Ausführungsformen umfasst dieses Modellierungsbauteil 16 auf einer sich verjüngenden Wandung 23 ein Außengewinde 22, das in ein an der inneren Wandung 28 des äußeren Rohres 21 ausgebildetes Innengewinde 20 einschraubbar ist.

Aufgrund der Ausbildung des Außengewindes 22 auf der geneigten bzw. sich verjüngenden Wandung 23 des Modellierungsbauteiles 16 ergibt sich beim Einschrauben dieses Bauteils in das Innengewinde 20 am äußeren Rohr 21 ein sich sukzessive erweiternder Gewindegang 24, der als Expansionskanal 12 fungiert. Dieser steht an einem Einlaufbereich 40 mit einem ebenfalls wieder durch eine Kryosondenspitze 34 gebildeten Umlenkkanal 38 in Fluidverbindung, so dass Kühlfluid über das innere Rohr 19 in Strömungsrichtung R_{S} expandierend durch den sich wendelförmig umlaufend erstreckenden Expansionskanal 12 strömen kann, um die Außenseite 7 der Kryosonde 2 abzukühlen.

Durch eine geeignete Wahl der Gewindesteigung und der Tiefe des jeweiligen Gewindegangs 24 kann hier zusätzlich auf die Kühlwirkung auf der Außenseite 7 Einfluss genommen werden.

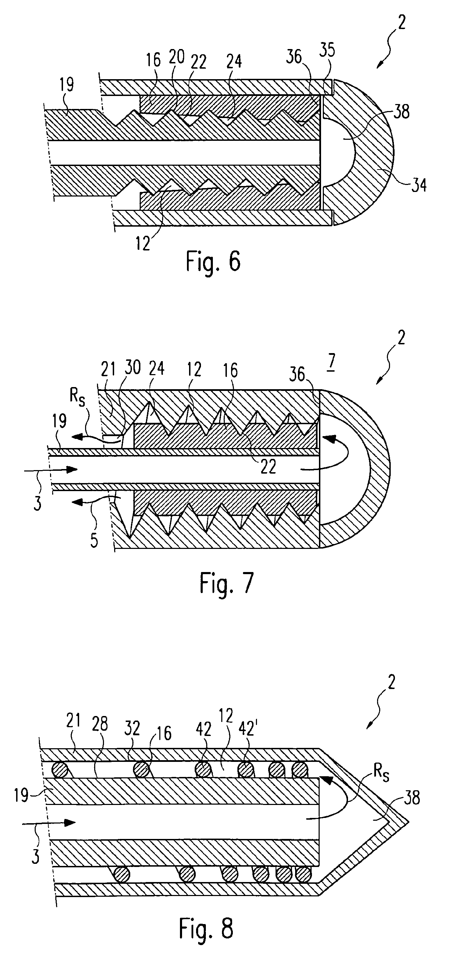

Fig. 6 zeigt eine fünfte Ausführungsform der Kryosonde 2. Im Gegensatz zur vorherigen Ausführungsform ist das Modellierungsbauteil 16 als ein sich erweiternder Hohlzylinder ausgebildet, der ein Innengewinde 20 aufweist, das derart komplementär zu einem Außengewinde 22 des inneren Rohres 18 ist, dass das Modellierungsbauteil 16 auf das Rohr 19 unter Bildung eines wendelförmig umlaufenden Expansionskanals 12 im Gewindegang 24 der beiden komplementären Gewinde 20, 22 aufschraubbar ist.

Darüber hinaus umfasst diese Ausführungsform der Kryosonde 2 eine Kryosondenspitze 34, die über einen Gewindebereich 35 auf ein freies Ende 36 des doppelwandigen Rohres 19, 21 derart aufschraubbar ist, dass die Größe des durch die Kryosondenspitze 34 gebildeten Umlenkkanals 38 durch ein heraus- bzw. hereinschrauben der Kryosondenspitze 34 anpassbar ist.

Fig. 7 zeigt eine sechste Ausführungsform der Kryosonde 2, umfassend wieder ein doppelwandiges Rohr 19, 21, wobei das innere Rohr 19 als Zulauf 3 und das äußere Rohr 21 bzw. der zwischen innerem Rohr 19 und äußerem Rohr 21 gebildete äußere Kanal 30 als Rücklauf 5 fungiert.

Im äußeren Kanal 30 ist zur Bildung eines Expansionskanals 12 ein Modellierungsbauteil 16 eingesetzt, das ein Außengewinde 22 aufweist. Das Rohr 21 weist ein dazu komplementäres Innengewinde 20 auf, das hier als ein so genanntes entgegen der Strömungsrichtung R_{S} verlaufendes kegeliges Gewinde ausgebildet ist. Das bedeutet, dass sich der Querschnitt des Gewindegangs 24 und somit der Expansionskanal 12 in Strömungsrichtung aufgrund dieses entgegen der Strömungsrichtung kegeliges Gewinde sukzessive vergrößert und so, wie schon in den zuvor beschriebenen Ausführungsformen, sukzessive erweitert. Natürlich wäre es hier auch möglich, an Stelle der gleichmäßigen Gewindesteigung eine zunehmende Gewindesteigung auszubilden oder aber auch das Modellierungsbauteil 16 entsprechend zu verjüngen, um beispielsweise eine in Richtung des freien Endes 36 der Kryosonde 2 stärker werdende Abkühlung der Außenseite 7 zu erzielen.

Natürlich ist es zudem möglich, statt dessen oder aber auch ergänzend an Stelle des entgegen der Strömungsrichtung R_{S} kegelig verlaufenden Gewindes 20 im Außenrohr 21 ein in Strömungsrichtung verlaufendes kegeliges Gewinde 22 im Modellierungsbauteil 16 anzuordnen.

Fig. 8 zeigt eine siebte Ausführungsform der Kryosonde 2, bei der zwischen einem als Zulauf 3 fungierenden inneren Rohr 19 und einem äußeren Rohr 21 an beiden Wandungen 28, 32 der Rohre 19, 21 ein wendelförmig um das innere Rohr 19 verlaufendes Modellierungsbauteil 16, in diesem Fall ein Draht, gewickelt ist, so dass sich zwischen den einzelnen Windungen 42, 42' und den jeweiligen Wandungen 28, 32 ein Expansionskanal 12 bildet, der ebenfalls wieder sich sukzessive erweiternd, wendelförmig um das Innenrohr 19 herumläuft. Ein über das innere Rohr 19 bzw. den Zulauf 3 in Strömungsrichtung R_{S} zufließende Kühlfluid tritt dann wieder über einen Umlaufkanal 38 in diesen wendelförmigen Expansionskanal 12 ein, wo es sich aufgrund des sich sukzessive vergrößernden Querschnitts schrittweise entspannt und abkühlt. Das Modellierungsbauteil 16 bzw. der Draht kann dabei sowohl ein eigenständig auf das innere Rohr 19 aufgewickeltes Bauteil als auch integral mit diesem und/oder dem äußeren Rohr 21 ausgebildetes Bauteil sein. Zudem ist es möglich, das Modellierungsbauteil 16 in entsprechenden Nuten oder dergleichen Führungseinrichtungen (nicht dargestellt) zu führen, um dessen Positionierung zu erleichtern.

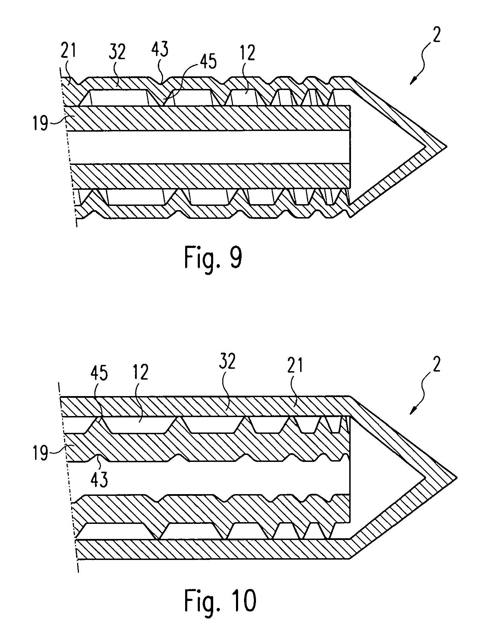

Fig. 9 zeigt eine achte Ausführungsform der erfindungsgemäßen Kryosonde 2, die nach demselben Prinzip funktioniert, wie die Ausführungsform aus Fig. 8. An Stelle des als Draht ausgeführten Modellierungsbauteils 16 ist hier eine entsprechende Einkerbungen 43 auf der äußeren Wandung 32 des äußeren Rohres 21 so angeordnet, dass sich die resultierenden Ausbuchtung 45 fluiddicht an das innere Rohr 19 presst. Da die Einkerbung 43 wendelförmig und mit zunehmender Steigung auf das äußere Rohr 21 aufgebracht ist, ergibt sich zwischen den einzelnen Windungen der Kerbe 43 bzw. der Ausbuchtung 45 ein umlaufender, sich sukzessiv erweiternder Expansionskanal 12 durch den das Gas entlang strömt und sich unter schrittweiser Abkühlung entspannt.

Eine ähnliche Ausführungsform zeigt Fig. 10, bei der die Einkerbungen 43 bzw. Ausbuchtungen 45 am inneren Rohr 19 angeordnet sind, so dass sich die Ausbuchtungen 45 unter Bildung eines entsprechenden Expansionskanals 12 fluiddicht an das äußere Rohr 21 bzw. dessen Wandung 32 anlegen.

Grundsätzlich sei angemerkt, dass natürlich neben den hier gezeigten Ausführungsformen des Modellierungsbauteils sämtliche Modellierungsbauteile verwendet werden können, die die Ausbildung eines sich sukzessive im Querschnitt vergrößernden Expansionskanals ermöglichen. Hier ist natürlich auch die Ausführung des Rohres bzw. des äußeren Rohres 21 als sich kegelig erweiterndes Rohr etc. denkbar.

Im Zusammenhang mit umlaufenden Gewindegängen zur Bildung des Expansionskanals können natürlich auch Doppel- und Mehrfachwendeln oder dergleichen mehrere sich insbesondere konzentrisch windende Gänge oder Kanäle etc. angewendet werden.

### Bezugszeichen

- 1: Kryochirurgisches Instrument
- 2: Kryosonde
- 3: Zulauf
- 4: Kühlgas
- 5: Ablauf
- 7: Außenseite
- 8: Leitungssystem
- 9: Kanalwandung
- 12: Expansionskanal
- 14: Wärmeleiter
- 16: Modellierungsbauteil
- 18: Rohr
- 19: inneres Rohr
- 20: Innengewinde
- 21: äußeres Rohr
- 22: Außengewinde
- 23: verjüngende Wandung
- 24: Gewindegang
- 26: innerer Kanal
- 28: innere Wandung
- 30: äußerer Kanal
- 32: äußere Wandung
- 34: Kryosondenspitze
- 36: freies Ende bzw. Rohrende
- 38: Umlenkkanal
- 40: Einlaufbereich
- 42: Windung
- 43: Einkerbung
- 44: Gasleitungseinrichtung
- 45: Ausbuchtung
- 46: Kryosondenhalter

## Patentansprüche

1. Kryochirurgisches Instrument, umfassend eine Kryosonde mit einem Kryosondengehäuse, mit einem von einem komprimierten Kühlfluid durchströmbaren Leitungssystem (8) zum Abkühlen wenigstens eines Teils der Kryosonde (2), weiter umfassend ein äußeres Rohr (21) und ein darin verlaufendes inneres Rohr (19) zur Zuführung des Kühlfluids wobei das äußere Rohr (21) das Kryosondengehäuse bildet oder über einen Wärmeleiter (14) mit dem Kryosondengehäuse verbunden ist,
**dadurch gekennzeichnet, dass** das Leitungssystem (8) einen Expansionskanal (12) aufweist, der einen sich in Strömungsrichtung (R_{S}) über eine vorbestimmte Länge sukzessive vergrößernden Leitungsquerschnitt derart aufweist, dass sich das komprimierte Kühlgas auf seinem Strömungsweg durch den Expansionskanal (12) sukzessive über die vorbestimmte Länge hinweg bei gleichzeitiger Abkühlung wenigstens teilweise entspannt, wobei der Expansionskanal (12) im Bereich der zu kühlenden Bereiche der Kryosonde (2) angeordnet und zwischen dem äußeren Rohr (21) und dem darin verlaufenden inneren Rohr (19) gebildet ist,

2. Kryochirurgisches Instrument nach Anspruch 1,
**dadurch gekennzeichnet, dass**
der Expansionskanal (12) über einen Wärmeleiter (14) mit der Außenseite (7) der Kryosonde (2) in wärmeleitfähiger Verbindung steht.

3. Kryochirurgisches Instrument nach Anspruch 2,
**dadurch gekennzeichnet, dass**
der Expansionskanal (12) einen in Abhängigkeit eines Wärmeübergangswiderstandes des Wärmeleiters (32) derart ausgebildeten Leitungsquerschnitt aufweist, dass sich auf der Außenseite (7) der Kryosonde (2) wenigstens über einen durch die vorbestimmte Länge definierten Teilbereich eine vorbestimmte Temperaturverteilung einstellt.

4. Kryochirurgisches Instrument nach einem der vorhergehenden Ansprüche,
**dadurch gekennzeichnet, dass**
der Expansionskanal (12) derart ausgebildet ist, dass das Kühlgas in einer turbulenten Strömung strömt.

5. Kryochirurgisches Instrument nach einem der vorhergehenden Ansprüche,
**dadurch gekennzeichnet, dass**
in wenigstens einem Teilbereich des Leitungssystem (8) zur Bildung des Expansionskanals (12) mindestens ein sich in Strömungsrichtung verjüngendes/erweiterndes Modellierungsbauteil (16) derart angeordnet ist, dass sich der resultierende Leitungsquerschnitt in Strömungsrichtung (R_{S}) sukzessive vergrößert.

6. Kryochirurgisches Instrument nach Anspruch 5,
**dadurch gekennzeichnet, dass**
der Expansionskanal (12) durch ein Rohr (21) und ein Modellierungsbauteil (16) gebildet wird, das als ein kegelstumpf oder dergleichen rotationssymmetrisches, sich in Strömungsrichtung (R_{S}) verjüngendes Bauteil in das Rohr (21) eingesetzt ist.

7. Kryochirurgisches Instrument nach Anspruch 5,
**dadurch gekennzeichnet, dass**
der Expansionskanal (12) durch ein Rohr (21) und ein Modellierungsbauteil (16) gebildet wird, das als ein sich in Strömungsrichtung (R_{S}) erweiterndes rotationssymmetrisches Hohlkörperbauteil in das Rohr (21) eingesetzt ist.

8. Kryochirurgisches Instrument nach Anspruch 6 oder 7,
**dadurch gekennzeichnet, dass**
das Rohr (21) wenigstens teilweise ein Kryosondengehäuse der Kryosonde (2) bildet.

9. Kryochirurgisches Instrument nach einem der Ansprüche 6 bis 8,
**dadurch gekennzeichnet, dass**
das Rohr (21) ein Innengewinde (20) und das Modellierungsbauteil (16) auf einer sich verjüngenden Wandung (23) ein komplementäres Außengewinde (22) aufweist, mit dem es in das Rohr (21) unter Bildung eines sich in Strömungsrichtung (R_{S}) sukzessive vergrößernden Expansionskanals (12) im resultierenden Gewindegang (24) einschraubbar ist.

10. Kryochirurgisches Instrument nach einem der Ansprüche 6 bis 9,
**dadurch gekennzeichnet, dass**
das Rohr (21) ein Innengewinde (20) und das Modellierungsbauteil (16) ein komplementäres Außengewinde (22) aufweist, mit dem es in das Rohr (21) unter Bildung des Expansionskanals (12) im resultierenden Gewindegang (24) einschraubbar ist, wobei das Außengewinde (22) als ein in Strömungsrichtung (R_{S}) verlaufendes kegeliges oder dergleichen Gewinde und/oder das Innengewinde (20) als ein in entgegengesetzter Richtung verlaufendes kegeliges oder dergleichen Gewinde ausgebildet ist.

11. Kryochirurgisches Instrument nach einem der vorhergehenden Ansprüche,
**gekennzeichnet durch**
eine Kryosondenspitze (34), die an einem Rohrende (36) angeordnet ist und das Innenrohr (19) mit dem Expansionskanal (12) unter Bildung eines Umlenkkanals (38) in Fluidverbindung bringt.

12. Kryochirurgisches Instrument nach Anspruch 11,
**dadurch gekennzeichnet, dass**
die Kryosondenspitze (34) derart am Rohrende (36) angeordnet ist, dass der Querschnitt des Umlenkkanals (38) veränderbar ist.

13. Kryochirurgisches Instrument nach einem der vorhergehenden Ansprüche,
**dadurch gekennzeichnet, dass**
das innere Rohr (19) ein Außengewinde (22) aufweist und das Modellierungsbauteil (16) als ein sich sukzessive erweiternder rotationssymmetrischer Hohlkörper ausgebildet ist, der an seiner innen liegenden Hohlkörperwandung ein Innengewinde (20) aufweist und mit diesem auf das Außengewinde (22) des inneren Rohres (19) unter Bildung des Expansionskanals (12) im Gewindegang (24) aufgeschraubt ist.

14. Kryochirurgisches Instrument nach einem der vorhergehenden Ansprüche,
**dadurch gekennzeichnet, dass**
um das innere Rohr (19) ein in Strömungsrichtung (R_{S}) wendelförmig, mit ansteigender Steigung verlaufendes Modellierungsbauteil (16), angeordnet ist, das mit den Wandungen (28,32) beider Rohre (19,21) jeweils in fluiddichter Verbindung steht, so dass zwischen benachbarten Windungen (42,42') und den Wandungen (26,32) ein wendelförmiger im Querschnitt sukzessive zunehmender Expansionskanal (12) gebildet wird.

15. Kryochirurgisches Instrument nach Anspruch 14,
**dadurch gekennzeichnet, dass**
das Modellierungsbauteil (16) integral am inneren Rohr (19) und/oder am äußeren Rohr (32) durch eine wendelförmige Einkerbung (43) der jeweiligen Wandung (28,32) des Rohres (19,21) gebildet ist, wobei in Strömungsrichtung (R_{S}) die Steigung der wendelförmigen Einkerbung (43) und/oder deren Breite (b) zunimmt.

## Claims

1. Cryosurgical instrument, comprising a cryoprobe with a cryoprobe housing, with a conduit system (8) through which a compressed cooling fluid can flow for cooling at least part of the cryoprobe (2), further comprising an outer tube (21) and, extending within the latter, an inner tube (19) for delivering the cooling fluid, wherein the outer tube (21) forms the cryoprobe housing or is connected to the cryoprobe housing via a heat conductor (14), **characterized in that** the conduit system (8) has an expansion channel (12) with a conduit cross section that gradually increases in size in the flow direction (R_{S}) over a predetermined length, in such a way that the compressed cooling gas, on its flow path through the expansion channel (12), gradually decompresses, at least in part, along the predetermined length and at the same time cools down, wherein the expansion channel (12) is arranged in the area of the areas of the cryoprobe (2) that are to be cooled and is formed between the outer tube (21) and the inner tube (19) extending within the latter.

2. Cryosurgical instrument according to Claim 1, **characterized in that** the expansion channel (12)
is connected in a thermally conductive manner to the outside (7) of the cryoprobe (2) via a heat conductor (14).

3. Cryosurgical instrument according to Claim 2, **characterized in that** the expansion channel (12) has a conduit cross section designed as a function of a heat transfer resistance of the heat conductor (32) in such a way that, on the outside (7) of the cryoprobe (2), a predetermined temperature distribution is established at least over a partial area defined by the predetermined length.

4. Cryosurgical instrument according to one of the preceding claims, **characterized in that** the expansion channel (12) is configured in such a way that the cooling gas flows in a turbulent flow.

5. Cryosurgical instrument according to one of the preceding claims, **characterized in that**, in order to form the expansion channel (12), at least one modelling component (16) that tapers/widens in the flow direction is arranged in at least a partial area of the conduit system (8), in such a way that the resulting conduit cross section gradually increases in size in the flow direction (R_{S}).

6. Cryosurgical instrument according to Claim 5, **characterized in that** the expansion channel (12) is formed by a tube (21) and a modelling component (16) that is inserted into the tube (21) as a frustoconical or similarly rotationally symmetrical component tapering in the flow direction (R_{S}).

7. Cryosurgical instrument according to Claim 5, **characterized in that** the expansion channel (12) is formed by a tube (21) and a modelling component (16) that is inserted into the tube (21) as a rotationally symmetrical hollow-body component that widens in the flow direction (R_{S}).

8. Cryosurgical instrument according to Claim 6 or 7, **characterized in that** the tube (21) at least partially forms a cryoprobe housing of the cryoprobe (2).

9. Cryosurgical instrument according to one of Claims 6 to 8, **characterized in that** the tube (21) has an internal thread (20) and the modelling component (16) has, on a tapering wall (23), a complementary external thread (22) with which it can be screwed into the tube (21) so as to form, in the resulting thread turn (24), an expansion channel (12) that gradually increases in size in the flow direction (R_{S}).

10. Cryosurgical instrument according to one of Claims 6 to 9, **characterized in that** the tube (21) has an internal thread (20) and the modelling component (16) has a complementary external thread (22) with which it can be screwed into the tube (21) so as to form the expansion channel (12) in the resulting thread turn (24), wherein the external thread (22) is configured as a conical or similar thread running in the flow direction (R_{S}) and/or the internal thread (20) is configured as a conical or similar thread running in the opposite direction.

11. Cryosurgical instrument according to one of the preceding claims, **characterized by** a cryoprobe tip (34), which is arranged on one tube end (36) and brings the inner tube (19) into fluid communication with the expansion channel (12) by forming a deflection channel (38).

12. Cryosurgical instrument according to Claim 11, **characterized in that** the cryoprobe tip (34) is arranged on the tube end (36) in such a way that the cross section of the deflection channel (38) can be modified.

13. Cryosurgical instrument according to one of the preceding claims, **characterized in that** the inner tube (19) has an external thread (22) and the modelling component (16) is configured as a rotationally symmetrical hollow body which gradually widens and which, on its inner hollow-body wall, has an internal thread (20) with which it can be screwed onto the external thread (22) of the inner tube (19) so as to form the expansion channel (12) in the thread turn (24).

14. Cryosurgical instrument according to one of the preceding claims, **characterized in that** a modelling component (16) extending in the shape of a helix and with an increasing pitch in the flow direction (R_{S}) is arranged around the inner tube (19) and is in fluid-tight connection with the walls (28, 32) of both tubes (19, 21), respectively, such that a helical expansion channel (12) increasing gradually in cross section is formed between adjacent windings (42, 42') and the walls (26, 32).

15. Cryosurgical instrument according to Claim 14, **characterized in that** the modelling component (16) is formed integrally on the inner tube (19) and/or on the outer tube (32) by a helical indentation (43) of the respective wall (28, 32) of the tube (19, 21), wherein the pitch of the helical indentation (43) and/or its width (b) increases in the flow direction (R_{S}).

## Revendications

1. Instrument cryochirurgical, comprenant une cryosonde avec un boîtier de cryosonde, avec un système de conduite (8) pouvant être parcouru par un fluide de refroidissement comprimé pour refroidir au moins une partie de la cryosonde (2), et comprenant en outre un tube extérieur (21) et un tube intérieur (19) s'étendant dans celui-ci, pour l'alimentation en fluide de refroidissement, le tube extérieur (21) formant le boîtier de la cryosonde ou étant connecté au boîtier de la cryosonde par le biais d'un conducteur thermique (14),
**caractérisé en ce que**
le système de conduite (8) présente un canal d'expansion (12) qui présente une section transversale de conduite augmentant progressivement sur une longueur prédéterminée dans la direction d'écoulement (R_{S}), de telle sorte que le gaz de refroidissement comprimé se détende au moins en partie sur son trajet d'écoulement à travers le canal d'expansion (12) progressivement sur la longueur prédéterminée avec refroidissement simultané, le canal d'expansion (12) étant disposé dans la région des régions à refroidir de la cryosonde (2) et étant formé entre le tube extérieur (21) et le tube intérieur (19) s'étendant dans celui-ci.

2. Instrument cryochirurgical selon la revendication 1,
**caractérisé en ce que**
le canal d'expansion (12) est en liaison thermoconductrice par le biais d'un conducteur thermique (14) avec le côté extérieur (7) de la cryosonde (2).

3. Instrument cryochirurgical selon la revendication 2,
**caractérisé en ce que**
le canal d'expansion (12) présente une section transversale de conduite réalisée en fonction d'une résistance de transition thermique du conducteur thermique (32) de telle sorte qu'une répartition prédéterminée de la température s'établisse sur le côté extérieur (7) de la cryosonde (2) au moins sur une région partielle définie par la longueur prédéterminée.

4. Instrument cryochirurgical selon l'une quelconque des revendications précédentes,
**caractérisé en ce que**
le canal d'expansion (12) est réalisé de telle sorte que le gaz de refroidissement s'écoule dans un écoulement turbulent.

5. Instrument cryochirurgical selon l'une quelconque des revendications précédentes,
**caractérisé en ce que**
dans au moins une région partielle du système de conduite (8) pour former le canal d'expansion (12) au moins un composant de modélisation (16) se rétrécissant/s'élargissant dans la direction d'écoulement est disposé de telle sorte que la section transversale de conduite résultante augmente progressivement dans la direction d'écoulement (R_{S}).

6. Instrument cryochirurgical selon la revendication 5,
**caractérisé en ce que**
le canal d'expansion (12) est formé par un tube (21) et un composant de modélisation (16) qui est inséré dans le tube (21) sous forme de composant tronconique ou similaire à symétrie de révolution,
se rétrécissant dans la direction d'écoulement (R_{S}).

7. Instrument cryochirurgical selon la revendication 5,
**caractérisé en ce que**
le canal d'expansion (12) est formé par un tube (21) et un composant de modélisation (16) qui est inséré dans le tube (21) sous forme de composant de type corps creux à symétrie de révolution s'élargissant dans la direction d'écoulement (R_{S}).

8. Instrument cryochirurgical selon la revendication 6 ou 7,
**caractérisé en ce que**
le tube (21) forme au moins en partie un boîtier de cryosonde pour la cryosonde (2).

9. Instrument cryochirurgical selon l'une quelconque des revendications 6 à 8,
**caractérisé en ce que**
le tube (21) présente un filetage interne (20) et le composant de modélisation (16) présente, sur une paroi se rétrécissant (23), un filetage externe complémentaire (22) avec lequel il peut être vissé dans le pas de filetage résultant (24) dans le tube (21) en formant un canal d'expansion (12) augmentant progressivement dans la direction d'écoulement (R_{S}).

10. Instrument cryochirurgical selon l'une quelconque des revendications 6 à 9,
**caractérisé en ce que**
le tube (21) présente un filetage interne (20) et le composant de modélisation (16) présente un filetage externe complémentaire (22) avec lequel il peut être vissé dans le pas de filetage résultant (24) dans le tube (21) en formant le canal d'expansion (12), le filetage externe (22) étant réalisé sous forme d'un filetage conique ou similaire s'étendant dans la direction d'écoulement (R_{S}) et/ou le filetage interne (20) étant réalisé sous forme d'un filetage conique ou similaire s'étendant dans la direction opposée.

11. Instrument cryochirurgical selon l'une quelconque des revendications précédentes,
**caractérisé par**
une pointe de cryosonde (34) qui est disposée à une extrémité du tube (36) et qui amène en liaison fluidique le tube intérieur (19) avec le canal d'expansion (12) en formant un canal de déviation (38).

12. Instrument cryochirurgical selon la revendication 11,
**caractérisé en ce que**
la pointe de cryosonde (34) est disposée à l'extrémité du tube (36) de telle sorte que la section transversale du canal de déviation (38) puisse être modifiée.

13. Instrument cryochirurgical selon l'une quelconque des revendications précédentes,
**caractérisé en ce que**
le tube intérieur (19) présente un filetage externe (22) et le composant de modélisation (16) est réalisé sous forme de corps creux à symétrie de révolution s'élargissant progressivement, qui présente, au niveau de sa paroi de corps creux située à l'intérieur, un filetage interne (20) et qui est vissé dans le pas de filetage (24) avec celui-ci sur le filetage externe (22) du tube intérieur (19) en formant le canal d'expansion (12).

14. Instrument cryochirurgical selon l'une quelconque des revendications précédentes,
**caractérisé en ce**
**qu'**autour du tube intérieur (19) est disposé un composant de modélisation (16) de forme hélicoïdale dans la direction d'écoulement (R_{S}), s'étendant avec une pente ascendante, qui est en liaison étanche aux fluides à chaque fois avec les parois (28, 32) des deux tubes (19, 21), de sorte qu'entre des spires adjacentes (42, 42') et les parois (26, 32) soit formé un canal d'expansion (12) de forme hélicoïdale dont la section transversale augmente progressivement.

15. Instrument cryochirurgical selon la revendication 14,
**caractérisé en ce que**
le composant de modélisation (16) est formé intégralement sur le tube intérieur (19) et/ou sur le tube extérieur (32) par un encochage de forme hélicoïdale (43) de la paroi respective (28, 32) du tube (19, 21), la pente de l'encochage de forme hélicoïdale (43) et/ou sa largeur (b) augmentant dans la direction d'écoulement (R_{S}).
